# FASCICULE DE BREVET EUROPEEN

(11) **EP 2 666 435 B1**
(45) Date de publication et mention de la délivrance du brevet: **31.01.2018**
(21) Numéro de dépôt: 13169084.4
(22) Date de dépôt: 24.05.2013
(51) Int. Cl.: A61B 46/00

(54) **Champ opératoire modulable, attache pour champ opératoire et procédé de fabrication associé**
Modulierbares Operationstuch, Befestigungselement für ein Operationstuch und entsprechendes Herstellungsverfahren
Adjustable surgical drape, attachment element for a surgical drape and related manufacturing method

(30) Priorité: 25.05.2012 FR 1254870
(43) Date de publication de la demande: 27.11.2013
(73) Titulaire: Vygon, 95440 Ecouen (FR)
(72) Inventeur: Lestoquoy, Patrick, 59551 ATTICHES (FR)
(74) Mandataire: Regimbeau

(56) Documents cités:
- EP-A1- 1 009 318
- WO-A2-2005/007020
- US-A- 4 600 001
- US-B1- 6 302 867
- "MediFilm 810", , 1 juin 2015 (2015-06-01), XP055192833, Extrait de l'Internet: URL:https://www.mylan.com/-/media/mylancom /files/products/transdermal/medifilm_810.p df [extrait le 2015-06-01]
- "polyester film Mylar Product Information Physical-Thermal Properties Typical Physical and Thermal Properties of Mylar Polyester Film Property Typical Value Unit Test Method Gauge and Type 92A End Use Industrial", , 30 juin 2003 (2003-06-30), XP055192838, Extrait de l'Internet: URL:http://usa.dupontteijinfilms.com/infor mationcenter/downloads/Physical_And_Therma l_Properties.pdf [extrait le 2015-06-01]

## Description

### DOMAINE DE L'INVENTION

L'invention concerne de manière générale les attaches pour champs opératoires et leurs procédés de fabrication.

Plus précisément, l'invention concerne les champs opératoires dissociables et/ou comprenant un élément de champ amovible.

### ARRIERE-PLAN TECHNOLOGIQUE

On connaît déjà de tels champs opératoires.

Par exemple, il existe des champs opératoires comprenant une fenêtre au travers de laquelle un opérateur peut accéder à un site opératoire pour introduire dans le corps un instrument tel qu'un tube ou un fil éventuellement prolongé par une ligne qui doit rester en place alors que le champ doit être retiré. Pour retirer le champ malgré la présence des instruments qui traversent la fenêtre, il est connu de découper le champ au moyen d'un outil coupant ou de le déchirer pour ouvrir le champ latéralement. Ces opérations compliquent cependant le travail de l'opérateur et constituent un risque pour les instruments et pour le patient.

Pour faciliter ces opérations, il a été proposé de munir le champ d'amorces ou de lignes de découpe ou de déchirement, comme décrit par exemple dans les documents EP 1 009 318 et WO 99/16377. Néanmoins, ces mesures ne facilitent que dans une mesure limitée le découpage du champ et ne résistent pas suffisamment à la pénétration des liquides, rendant ainsi possibles des fautes d'aseptie et des infections.

On a donc proposé dans la demande de brevet WO 2007/083032 un champ opératoire comprenant deux parties de feuille maintenues dans le prolongement l'une de l'autre par un ruban adhésif imperméable. Le champ ainsi obtenu résiste à la pénétration des liquides et peut être pelé par l'opérateur lorsque celui-ci souhaite ouvrir le champ, sans retirer les instruments en place.

La mise en oeuvre d'un tel ruban permet de rendre la jonction entre les champs imperméable aux liquides, et résiste aux tests actuels de pénétration de liquide basés sur colonne d'eau. Toutefois, le pelage du ruban adhésif par l'opérateur laisse des résidus collants sur le champ, ce qui peut gêner l'opérateur au cours de l'opération. L'adhésif peut alors se transférer sur le cathéter en place au cours de l'opération, risquant ainsi son extraction accidentelle. Il faut alors remettre le cathéter en place, ce qui peut être dangereux pour le patient.

Le document WO 2009/010509 quant à lui propose un champ opératoire comprenant deux films pelables imperméables dont deux bords sont jointifs. Les films sont maintenus ensemble par un ruban étroit pelable imperméable, via une soudure thermique permettant de séparer ultérieurement les films et la bande par pelage. Le film et le ruban sont formés de deux feuilles en polyéthylène (PE) superposées, dont l'une est en polyéthylène à très basse densité (PEBD). La soudure thermique est alors réalisée de sorte que la feuille en PEBD des films se trouve en contact avec la feuille en PE du ruban. En variante, la soudure thermique est réalisée alors que la feuille en PEBD des films est en contact avec la feuille en PEBD du ruban. La soudure thermique fusionne les feuilles en PEBD qui adhèrent entre elles et, lors d'un pelage, leur très faible résistance mécanique permet l'ouverture du champ opératoire par un opérateur.

Les champs opératoires ainsi obtenus sont imperméables et résistent à la pénétration des liquides lors de leur utilisation.

Toutefois, leur procédé de fabrication peut présenter des difficultés. Par exemple, la soudure du ruban étroit sur les parties homologues du champ opératoire nécessite de la main d'oeuvre et un geste très précis engendrant pertes et investissements en matériel de soudure. La réalisation du champ en tout ou partie dans un film dissociable est en outre coûteuse en raison du prix de la matière première utilisée. Par ailleurs, lorsque le champ est composé de plusieurs matériaux différents, par exemple lorsqu'il comporte une fenêtre transparente en polyéthylène (PE) encadrée d'une feuille en non tissé, la soudure du ruban est délicate en raison des changements d'épaisseurs et de passage d'un matériau à un autre.

La soudure thermique des parties de champ opératoire au moyen du ruban pelable a donc un coût de revient élevé en termes de matière et de procédé de fabrication et peut se révéler impossible selon les matières utilisées (notamment dans le cas des champs opératoire formés dans des matières qui ne sont pas thermiquement soudables).

Enfin, le document EP 2 364 667 décrit un champ opératoire, notamment pour accouchement, comprenant une poche amovible fixée sur le champ par thermoscellage d'un film pelable multicouche en polyéthylène. De la sorte, après remplissage de la poche de recueil, celle-ci est retirée du champ par déchirement de la couche inférieure du film dissociable. Le document WO2005/007020 A2 décrit un dispositif adhésif utilisé comme pansement, dispositif d'administration d'ingrédient ou dispositif de fixation intraveineux (voir notamment la figure 14).

### RESUME DE L'INVENTION

L'invention concerne une attache de champ adaptée pour être utilisée avec un champ opératoire tel que décrit dans la première revendication indépendante, ainsi que son procédé de fabrication tel que décrit dans la revendication 14. Un objectif de l'invention est de proposer un champ opératoire modulable, adapté le cas échéant pour être séparé en deux parties sans l'aide d'un instrument coupant, tout en garantissant son imperméabilité et sans pour autant le fragiliser, qui soit en outre simple à réaliser, d'un coût modéré, et soit adapté pour une fabrication en ligne, quel(s) que soi(en)t son (ses)matériau(x) constitutif(s).

Un autre objectif de l'invention est de proposer un champ opératoire capable de recevoir de manière amovible un élément de champ séparé, de manière rapide et sûre.

Pour cela, un aspect de l'invention propose un champ opératoire comprenant une feuille principale, caractérisé en ce qu'il comprend une attache de champ comprenant :
- un film multicouche dissociable présentant au moins une couche interne et une couche externe, la couche interne présentant une résistance mécanique plus faible que la couche externe de sorte que la couche interne rompt ou se déchire avant la couche externe lors d'un effort de traction exercé sur le film multicouche, et
- une couche d'adhésif, fixée sur la couche interne du film multicouche dissociable,
l'attache de champ étant fixée sur la feuille principale par l'intermédiaire de la couche d'adhésif.

D'autres caractéristiques optionnelles et non limitatives du champ opératoire sont les suivantes :
* une cohésion interne de la couche interne est plus faible qu'une cohésion interne de la couche externe ;
* la couche interne est plus fine que la couche externe ;
* il comprend en outre une couche support soudée sur la couche interne du film dissociable, sur laquelle est fixée la couche d'adhésif ;
* la couche interne du film dissociable comprend l'un au moins des matériaux suivants : du polyéthylène basse densité (PEBD), du polyéthylène très basse densité (PETBD), du polyéthylène/éthyle vinyle acétate (PE/EVA) et la couche externe comprend l'un au moins des matériaux suivants : du polyéthylène (PE) du polyamide (PA), du poly(téréphtalate d'éthylène) (PET).
* l'attache comprenant en outre une couche supplémentaire d'adhésif (28) fixée sur le film dissociable, du côté opposé de la couche externe ;
* la couche supplémentaire d'adhésif est recouverte par une feuille de protection pelable ;
* la feuille principale est divisée en deux parties de feuille qui sont maintenues dans le prolongement l'une de l'autre par l'attache de champ (20) de manière à former la feuille principale dudit champ ; et
* la couche d'adhésif est appliquée localement en deux zones distinctes et séparées du film dissociable, et l'attache de champ est fixée sur les parties de feuilles de la feuille principale de sorte que chacune desdites zones soit fixée sur l'une des parties de feuille.

Selon un deuxième aspect, l'invention concerne également une attache pour un champ opératoire comme décrit ci-dessus, laquelle comprend un film multicouche dissociable présentant au moins une couche interne et une couche externe, la couche interne présentant une résistance mécanique plus faible que la couche externe de sorte que la couche interne rompt ou se déchire avant la couche externe lors d'un effort de traction exercé sur le film multicouche, et une couche d'adhésif, fixée sur la couche interne du film dissociable.

D'autres caractéristiques optionnelles et non limitatives de l'attache pour champ opératoire sont les suivantes :
* elle comprend en outre une feuille de protection adaptée pour recouvrir la couche d'adhésif ; et
* la couche d'adhésif est appliquée localement en deux zones distinctes et séparées du film dissociable.

Selon un troisième aspect, l'invention propose également un procédé de fabrication d'un champ opératoire comme décrit ci-dessus, comprenant les étapes suivantes :
- fabriquer l'attache de champ, et
- fixer la couche d'adhésif de l'attache de champ sur le champ opératoire.

De manière non limitative, la couche d'adhésif peut être appliquée en deux zones distinctes et séparées du film dissociable au cours de la fabrication de l'attache de champ, et le procédé peut en outre comprend, après l'étape de fixation de l'attache de champ sur ledit champ opératoire , une étape de découpe de la feuille principale du champ opératoire en deux parties de feuilles maintenues dans le prolongement l'une de l'autre par l'attache de champ.

### BREVE DESCRIPTION DES DESSINS

D'autres caractéristiques, buts et avantages de l'invention apparaîtront mieux à la lecture de la description détaillée qui va suivre, faite en référence aux figures annexées données à titre d'exemples non limitatifs et sur lesquels :

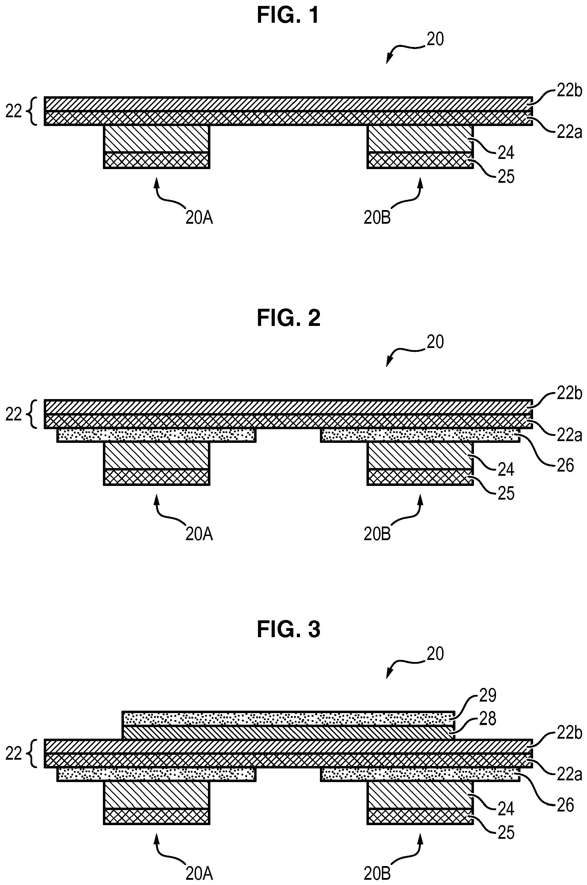

- la figure 1 illustre un premier exemple de réalisation d'une attache de champ opératoire conforme à l'invention ;
- la figure 2 illustre un deuxième exemple de réalisation d'une attache de champ opératoire conforme à l'invention ;
- la figure 3 illustre un troisième exemple de réalisation d'une attache de champ opératoire conforme à l'invention ; et

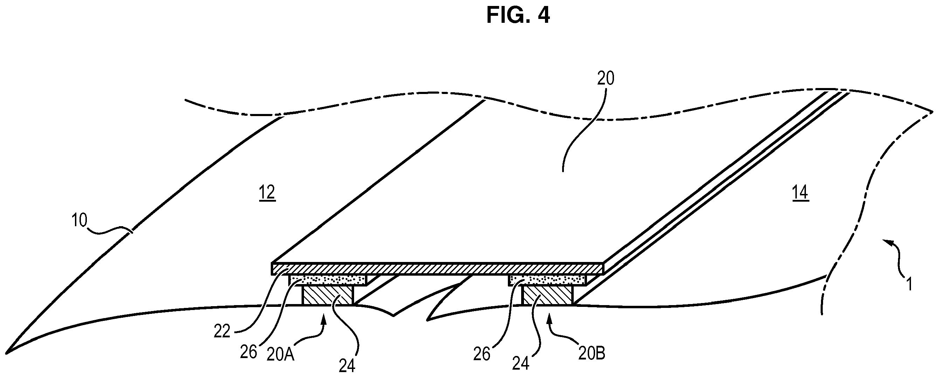

- la figure 4 illustre un exemple de réalisation d'un champ opératoire conforme à l'invention.

### DESCRIPTION DETAILLEE D'UN MODE DE REALISATION

Un champ opératoire 1 conforme à la présente invention comprend une feuille principale 10, sur laquelle est fixée une attache de champ opératoire 20. L'attache 20 est adaptée pour maintenir ensemble deux parties de champ 12, 14 dans le prolongement l'une de l'autre de manière à former la feuille principale 10, et/ou fixer un élément de champ de manière amovible.

L'attache de champ 20 peut être rapportée sur le champ opératoire 1 lors de sa fabrication.

Elle comprend un film dissociable 22, sur lequel est fixée une couche d'adhésif 24.

Le film dissociable 22 comprend un film multicouches, comprenant au moins une couche interne 22a destinée à recevoir la couche d'adhésif 24, et une couche externe 22b. Le film multicouches 22 peut par exemple être obtenu par coextrusion de ses couches.

La couche interne 22a est adaptée pour être déchirée par un opérateur souhaitant retirer l'attache de champ 20. Plus précisément, la couche interne 22a présente une plus faible résistance mécanique en traction que la couche externe 22b, de manière à rompre ou à se déchirer avant la couche externe 22b lors d'un effort de traction exercé sur le film multicouche 22 dans une direction normale à la direction d'extension desdites couches, la couche externe 22b restant intacte ou ne subissant qu'une faible décohésion interne. Pour cela, la couche interne 22a peut être réalisée dans un matériau présentant une cohésion interne plus faible que la cohésion interne de la couche externe 22b et éventuellement présenter une épaisseur (dans la direction normale au plan des couches) plus faible.

Par exemple, pour une couche externe 22b en polyéthylène (PE), la couche interne 22a peut notamment comprendre du polyéthylène basse densité (PEBD), du polyéthylène très basse densité (PETBD), du polyéthylène/Ethyle Vinyle Acétate (PE/EVA), ou tout autre matériau déchirable adapté présentant une faible cohésion interne.

En variante, la couche externe peut être réalisée en polyamide (PA) ou en poly(téréphtalate d'éthylène) (PET), ou tout autre polymère adapté pour la réalisation de champs opératoires.

Le film dissociable 22 peut comprendre en outre d'autres couches, selon les propriétés mécaniques désirées. Par exemple le film dissociable 22 peut comprendre une ou plusieurs couches en plus des couches interne et externe, ayant une résistance mécanique plus importante que la couche interne 22a, et réalisées par exemple en polyéthylène (PE).

De manière optionnelle, le film dissociable 22 peut être symétrique afin de pouvoir être utilisé dans n'importe quel sens sans risquer de coller la couche d'adhésif sur une couche ayant une résistance mécanique trop élevée, et limiter ainsi les erreurs potentielles de fabrication du champ opératoire 1. Par exemple, le film dissociable 22 peut comprendre une couche interne 22a réalisé dans un matériau à faible cohésion mécanique compris dans la liste ci-dessus, une ou plusieurs couches externes, fixées sur la couche interne 22a, comprenant par exemple du PE, et une couche supérieure de recouvrement, identique à la couche interne 22a, disposée sur les couches externes.

La couche d'adhésif 24 est choisie de manière à présenter une résistance au pelage plus importante que la résistance mécanique de la couche interne 22a du film dissociable 22, de sorte que lorsqu'un effort de traction est appliqué sur l'attache 20, la couche interne 22a du film dissociable qui rompt avant que l'adhésif ne se décolle. En effet, la rupture entre le champ opératoire 1 et l'attache 20 doit se faire au niveau de la couche interne 22a du film dissociable 22, et non de la couche d'adhésif 24 ni du champ opératoire 1, afin de surmonter les inconvénients précités de l'art antérieur. Par exemple, la couche d'adhésif 24 présente une tenue mécanique d'au moins deux Newtons pour une épaisseur d'un pouce (correspondant environ à 2,54 cm). Ce point sera détaillé plus avant dans la suite de cette description.

Par ailleurs, la couche d'adhésif 24 doit être adaptée pour être fixée directement ou indirectement sur la couche interne 22a du film dissociable 22. Ainsi, la couche d'adhésif 24 peut être fixée directement sur la couche interne 22a, ou par l'intermédiaire d'une couche support 26.

Par exemple, la couche d'adhésif 24 peut être fixée sur une feuille de polyéthylène, la feuille de polyéthylène 26 étant alors soudée sur la couche interne 22a du film dissociable 22. Cette forme de réalisation présente l'avantage de permettre de rapporter la couche d'adhésif 24 préalablement fixée sur la feuille de polyéthylène puis de simplement souder la feuille de polyéthylène sur la couche interne 22a du film dissociable 22. On pourra notamment utiliser un adhésif du type sensible à la pression (connus généralement sous l'acronyme anglais PSA pour Pressure-Sensitive Adhesive).

L'attache de champ 20 ainsi obtenue peut ensuite être fixée sur un champ opératoire 1, puis pelée manuellement par un opérateur lorsque celui-ci le juge nécessaire. Lors du pelage, l'attache de champ 20 rompt alors au niveau de sa couche interne 22a, laissant la couche d'adhésif 24 recouverte par une partie de la couche interne 22a sur le champ (et le cas échéant de la couche support 26), de sorte que le champ opératoire 1 ne colle pas.

Selon une forme de réalisation préférée, le champ opératoire 1 est réalisé dans un matériau non absorbant afin de limiter les risques d'infection au niveau de l'interface entre l'attache de champ 20 et le champ opératoire 1.

Un exemple de fabrication d'un champ dissociable va à présent être décrit.

Au cours d'une première étape, une attache de champ 20 est fabriquée. Avantageusement, l'attache de champ 20 peut être réalisée de manière automatique par une installation adaptée, en empilant et en fixant ensemble le film dissociable 22 et la couche d'adhésif 24, par l'intermédiaire ou non de la couche support 26, les différents éléments étant amenés à partir d'autant de rouleaux. Il est ainsi possible de réaliser des bobines ou de longues bandes d'attache de champ 20 de manière rapide et économique, qui peuvent être stockées avant leur fixation au champ 1.

Dans le cas de la réalisation d'un champ dissociable, la couche d'adhésif 24 et le cas échéant la couche support 26 sont appliquées en deux zones séparées et distinctes 20A, 20B de la couche interne 22a du film dissociable 22, par exemple selon deux bandes sensiblement parallèles.

Les attaches de champ 20 ainsi obtenues peuvent alors être stockées et/ou transportées avant leur utilisation. A cette fin, il est alors possible de recouvrir la surface libre des couches adhésives d'une feuille de protection 25, par exemple un papier siliconé. La feuille de protection 25 permet d'éviter les risques d'endommagement des couches d'adhésif, ainsi que leur adhésion intempestive. La feuille de protection 25 doit alors être retirée avant fixation de l'attache 20 sur le champ 1.

En variante, la couche d'adhésif 24 est activable, par exemple par pression, par application d'ultraviolets, de chaleur, etc. et n'adhère donc qu'après activation.

L'attache de champ 20 est ensuite amenée sur une feuille principale 10 d'un champ opératoire 1 mono- ou multi-matériaux, et fixée sur celle-ci à l'endroit désiré. Par exemple, l'attache 20 peut s'étendre d'un bord à l'autre de la feuille principale 10, de manière à la séparer en deux parties distinctes 12, 14. En variante, l'attache 20 peut s'étendre sur une partie seulement de la longueur de la feuille principale 10.

La feuille principale 10 du champ peut ensuite être découpée entre les zones distinctes et séparées de l'attache de champ 20, par exemple manuellement à l'aide de ciseaux ou de manière automatique à l'aide d'une lame, de manière à séparer la feuille principale 10 en deux parties de feuille 12, 14, sans endommager toutefois le film dissociable 22. La présence du film dissociable 22 permet d'empêcher la pénétration de liquides au travers du champ 1, malgré la séparation de la feuille principale 10 en deux parties de feuille, tout en permettant la dissociation des deux parties de feuilles 12, 14 suite au pelage de l'attache 20.

En variante, la feuille principale 10 peut être composée de deux parties de feuille 12, 14 distinctes avant fixation de l'attache de champ 20, soit parce que la feuille principale 10 a été découpée préalablement, soit parce que les parties de feuille 12, 14 ont été réalisées séparément. Les parties de feuille 12, 14 sont alors positionnées en regard, puis l'attache de champ 20 est fixée sur celles-ci de telle sorte que chaque partie de feuille 12, 14 soit fixée à l'une des zones 20A, 20B de l'attache 20.

Dans tous les cas, la mise en oeuvre d'une attache 20 conforme à l'invention permet de fixer ensemble des parties de champ quel(s) que soi(en)t leur(s) matériau(x) respectif(s) de manière simple, rapide et efficace. Aucune étape de soudage n'est nécessaire, seule l'application de l'adhésif sur la partie de champ à rendre dissociable doit être réalisée.

Par ailleurs, les attaches de champ 20 peuvent être fabriquées en ligne sur une machine adaptée, par superposition et fixation du film dissociable 22, de la couche d'adhésif 24 et des éventuelles couches support 26 et de protection 25. En variante, l'ensemble des couches formant le film dissociable 22 et la couche d'adhésif 24 peut être coextrudé, puis superposé et fixé aux couches support 26 et de protection 25.

Seule l'étape de fixation de l'attache de champ 20 est donc réalisée à part, soit manuellement, soit par une machine adaptée. Or cette étape, rapide est simple à réaliser, consiste simplement à retirer l'éventuelle feuille de protection 25, à coller l'attache sur une feuille de champ puis à découper le champ opératoire 1 sous l'attache de champ 20. Le procédé de fabrication des champs pelables est donc semi-automatisé, ce qui permet d'augmenter la cadence de fabrication, tout en limitant les coûts de matière et de main d'oeuvre.

L'attache de champ 20 peut également être utilisée de manière à fixer un élément de champ amovible, tel qu'une poche de recueil.

Pour cela, l'attache de champ 20 comprend en outre, au niveau de sa face externe, correspondant à la couche externe 22b du film dissociable 22, une bande d'adhésif supplémentaire 28. Cette bande d'adhésif supplémentaire 28 peut être recouverte d'une feuille de protection 29, telle qu'un papier siliconé, lorsque l'élément de champ n'est fixé pas sur le champ opératoire 1 au moment de sa fabrication mais ultérieurement.

L'élément amovible peut être fixé sur la bande d'adhésif supplémentaire 28, après avoir retiré l'éventuelle feuille de protection 29, tandis que la face interne de l'attache 20 est fixée sur la feuille principale 10 du champ opératoire 1.

La bande d'adhésif supplémentaire 28 est choisie de manière à être capable de supporter la charge de l'élément de champ amovible sans rompre, et présente une résistance mécanique supérieure à celle de la couche interne 22a du film dissociable 22 afin de garantir que la rupture de l'attache 20 lors du pelage de l'élément de champ se fasse au niveau de la couche interne 22a pour ne pas laisser de traces d'adhésif sur le champ 1. Par exemple, la couche d'adhésif 24 supplémentaire 28 peut être identique à la couche d'adhésif 24 fixée sur la couche interne 22a.

Bien entendu, l'attache peut être fixée sur le champ opératoire 1 avant ou après fixation à l'élément amovible.

Lorsque l'opérateur souhaite retirer l'élément amovible, il suffit alors de peler l'attache 20 en tirant sur l'élément. L'attache de champ 20 rompt et se sépare au niveau de la couche interne 22a du film dissociable 22, laissant la couche d'adhésif 24 (munie le cas échéant de la couche support 26) et une partie de la couche interne 22a sur le champ opératoire 1, le reste restant accroché à l'élément amovible.

Dans cette forme de réalisation, l'attache de champ 20 peut comprendre une unique zone munie d'adhésifs.

Dans une variante de réalisation, l'attache de champ 20 peut à la fois rendre le champ dissociable et fixer de manière amovible un élément de champ, la couche d'adhésif 24 côté couche interne 22a étant alors répartie en deux zones distinctes et séparées, chacune des zones étant prévue pour maintenir deux parties de feuilles 12, 14 dans le prolongement l'une de l'autre.

## Revendications

1. Attache (20) de champ adaptée pour être utilisée avec un champ opératoire (1), ladite attache (20) comprenant :
- un film multicouche dissociable (22) présentant au moins une couche interne (22a) et une couche externe (22b), la couche interne (22a) présentant une résistance mécanique plus faible que la couche externe (22b), de sorte que la couche interne (22a) rompt ou se déchire avant la couche externe (22b) lors d'un effort de traction exercé sur le film multicouche (22) dans une direction normale à la direction d'extension desdites couches (22a, 22b), et
- une couche d'adhésif (24), fixée sur la couche interne (22a) du film dissociable (22), ladite couche d'adhésif (24) présentant une résistance au pelage plus importante que la résistance mécanique de la couche interne (22a) du multicouche dissociable (22).

2. Attache (20) de champ selon la revendication 1, comprenant en outre une feuille de protection adaptée pour recouvrir la couche d'adhésif (24).

3. Attache (20) selon l'une des revendications 1 ou 2, dans laquelle la couche d'adhésif (24) est appliquée localement en deux zones distinctes et séparées (20A, 20B) du film dissociable (22).

4. Attache (20) selon l'une des revendications 1 à 3, dans laquelle une cohésion interne de la couche interne (22a) est plus faible qu'une cohésion interne de la couche externe (22b).

5. Attache (20) selon l'une des revendications 1 à 4, dans laquelle la couche interne (22a) est plus fine que la couche externe (22b).

6. Attache (20) selon l'une des revendications 1 à 5, comprenant en outre une couche support (26) soudée sur la couche interne (22a) du film dissociable (22), sur laquelle est fixée la couche d'adhésif (24).

7. Attache (20) selon l'une des revendications 1 à 6, dans laquelle la couche interne (22a) du film dissociable (22) comprend l'un au moins des matériaux suivants : du polyéthylène basse densité (PEBD), du polyéthylène très basse densité (PETBD), du polyéthylène/éthyle vinyle acétate (PE/EVA).

8. Attache (20) selon l'une des revendications 1 à 7, dans laquelle la couche externe (22b) comprend l'un au moins des matériaux suivants : du polyéthylène (PE) du polyamide (PA), du poly(téréphtalate d'éthylène) (PET).

9. Attache (20) selon l'une des revendications 1 à 8, dans laquelle l'attache comprend en outre une couche supplémentaire d'adhésif (28) fixée sur le film dissociable (22), du côté de la couche externe (22b).

10. Attache (20) selon la revendication 9, dans laquelle la couche supplémentaire d'adhésif (28) est recouverte par une feuille de protection pelable (29).

11. Champ opératoire (1) comprenant une feuille principale (10) et une attache de champ (20) selon l'une des revendications 1 à 10.

12. Champ opératoire (1) selon la revendication 11, dans lequel la feuille principale (10) est divisée en deux parties de feuille (12, 14) qui sont maintenues dans le prolongement l'une de l'autre par l'attache de champ (20) de manière à former la feuille principale (10) dudit champ (1).

13. Champ opératoire (1) selon la revendication 12, dans lequel la couche d'adhésif (24) est appliquée localement en deux zones distinctes et séparées (20A, 20B) du film dissociable (22), et l'attache de champ (20) est fixée sur les parties de feuilles (12, 14) de la feuille principale (10) de sorte que chacune desdites zones soit fixée sur l'une des parties de feuille (12, 14).

14. Procédé de fabrication d'un champ opératoire (1) selon l'une des revendications 11 à 13, comprenant les étapes suivantes :
- fabriquer une attache de champ (20) selon l'une des revendications 1 à 10, et
- fixer la couche d'adhésif (24) de l'attache de champ (20) sur la feuille principale (10) du champ opératoire (1).

15. Procédé de fabrication selon la revendication 14, dans lequel on applique la couche d'adhésif (24) en deux zones distinctes et séparées du film dissociable (22) au cours de la fabrication de l'attache de champ (20), et le procédé comprend en outre, après l'étape de fixation de l'attache de champ (20) sur ledit champ opératoire (1), une étape de découpe de la feuille principale (10) du champ opératoire (1) en deux parties de feuilles (12, 14) maintenues dans le prolongement l'une de l'autre par l'attache de champ (20).

## Patentansprüche

1. Tuchbefestigung (20) für die Verwendung mit einem chirurgischen Abdecktuch (1), wobei die Befestigung (20) Folgendes umfasst:
- eine trennbare Mehrschichtenfolie (22), die zumindest eine Innenschicht (22a) und eine Außenschicht (22b) aufweist, wobei die Innenschicht (22a) eine schwächere mechanische Festigkeit als die Außenschicht (22b) aufweist, so dass die Innenschicht (22a) bei einer auf die Mehrschichtenfolie (22) in einer zur Erstreckungsrichtung der Schichten (22a, 22b) normalen Richtung ausgeübten Zugkraft vor der Außenschicht (22b) reißt oder zerreißt, und
- eine Klebstoffschicht (24), die auf der Innenschicht (22a) der trennbaren Folie (22) befestigt ist, wobei die Klebstoffschicht (24) eine stärkere Abschälfestigkeit als die mechanische Festigkeit der Innenschicht (22a) der trennbaren Mehrschichtenfolie (22) aufweist.

2. Tuchbefestigung (20) nach Anspruch 1, die ferner eine Schutzfolie umfasst, die geeignet ist, die Klebstoffschicht (24) abzudecken.

3. Befestigung (20) nach einem der Ansprüche 1 oder 2, wobei die Klebstoffschicht (24) örtlich in zwei unterschiedlichen und getrennten Bereichen (20A, 20B) der trennbaren Folie (22) aufgebracht wird.

4. Befestigung (20) nach einem der Ansprüche 1 bis 3, wobei ein innerer Zusammenhalt der Innenschicht (22a) schwächer als ein innerer Zusammenhalt der Außenschicht (22b) ist.

5. Befestigung (20) nach einem der Ansprüche 1 bis 4, wobei die Innenschicht (22a) dünner als die Außenschicht (22b) ist.

6. Befestigung (20) nach einem der Ansprüche 1 bis 5, die ferner eine Trägerschicht (26) umfasst, die auf die Innenschicht (22a) der trennbaren Folie (22) geschweißt ist, auf der die Klebstoffschicht (24) befestigt ist.

7. Befestigung (20) nach einem der Ansprüche 1 bis 6, wobei die Innenschicht (22a) der trennbaren Folie (22) zumindest einen der folgenden Stoffe umfasst: Polyethylen mit niedriger Dichte (LDPE), Polyethylen mit sehr niedriger Dichte (VLDPE), Polyethylen/Ethylenvinylacetat (PE/EVA).

8. Befestigung (20) nach einem der Ansprüche 1 bis 7, wobei die Außenschicht (22b) zumindest einen der folgenden Stoffe umfasst: Polyethylen (PE), Polyamid (PA), Polyethylenterephthalat (PET).

9. Befestigung (20) nach einem der Ansprüche 1 bis 8, wobei die Befestigung ferner eine zusätzliche Klebstoffschicht (28) umfasst, die auf der trennbaren Folie (22) außenschichtseitig (22b) befestigt ist.

10. Befestigung (20) nach Anspruch 9, wobei die zusätzliche Klebstoffschicht (28) mit einer abschälbaren Schutzfolie (29) abgedeckt ist.

11. Chirurgisches Abdecktuch (1), das eine Hauptfolie (10) und eine Tuchbefestigung (20) nach einem der Ansprüche 1 bis 10 umfasst.

12. Chirurgisches Abdecktuch (1) nach Anspruch 11, wobei die Hauptfolie (10) in zwei Folienteile (12, 14) aufgeteilt ist, die in der jeweiligen Verlängerung durch die Tuchbefestigung (20) so gehalten werden, dass sie die Hauptfolie (10) des Abdecktuchs (1) bilden.

13. Chirurgisches Abdecktuch (1) nach Anspruch 12, wobei die Klebstoffschicht (24) örtlich in zwei unterschiedlichen und getrennten Bereichen (20A, 20B) der trennbaren Folie (22) aufgebracht ist und die Tuchbefestigung (20) auf den Folienteilen (12, 14) der Hauptfolie (10) so befestigt ist, so dass jeder der Bereiche auf einem der Folienteile (12, 14) befestigt ist.

14. Verfahren zur Herstellung eines chirurgischen Abdecktuchs (1) nach einem der Ansprüche 11 bis 13, das die folgenden Schritte umfasst:
- eine Tuchbefestigung (20) nach einem der Ansprüche 1 bis 10 herstellen; und
- die Klebstoffschicht (24) der Tuchbefestigung (20) auf der Hauptfolie (10) des chirurgischen Abdecktuchs (1) befestigen.

15. Verfahren zur Herstellung nach Anspruch 14, wobei die Klebstoffschicht (24) im Laufe der Herstellung der Tuchbefestigung (20) in zwei unterschiedlichen und getrennten Bereichen der trennbaren Folie (22) aufgebracht wird, und das Verfahren umfasst ferner nach dem Schritt mit der Befestigung der Tuchbefestigung (20) auf dem chirurgischen Abdecktuch (1) einen Schritt mit Zuschnitt der Hauptfolie (10) des chirurgischen Abdecktuchs (1) in zwei Folienteile (12, 14), die in der jeweiligen Verlängerung durch die Tuchbefestigung (20) gehalten werden.

## Claims

1. A drape attachment (20) adapted for using with a surgical drape (1), said attachment (20) comprising:
- a separable multilayer film (22) having at least one internal layer (22a) and an external layer (22b), the internal layer (22a) having lower mechanical resistance than the external layer (22b), such that the internal layer (22a) breaks or tears before the external layer (22b) during traction force exerted on the multilayer film (22) in a direction normal to the direction of extension of said layers (22a, 22b), and
- a layer of adhesive (24), fixed to the internal layer (22a) of the separable film (22), said layer of adhesive (24) having greater resistance to peeling than the mechanical resistance of the internal layer (22a) of the separable film (22).

2. The drape attachment (20) according to claim 1, further comprising a protective sheet adapted to cover the layer of adhesive (24).

3. The attachment (20) according to any of claims 1 or 2, wherein the layer of adhesive (24) is applied locally in two distinct and separate zones (20A, 20B) of the separable film (22).

4. The attachment (20) according to any of claims 1 to 3, wherein internal cohesion of the internal layer (22a) is weaker than internal cohesion of the external layer (22B).

5. The attachment (20) according to any of claims 1 to 4, wherein the internal layer (22a) is finer than the external layer (22b).

6. The attachment (20) according to any of claims 1 to 5, further comprising a support layer (26) welded onto the internal layer (22a) of the separable film (22), on which the layer of adhesive (24) is fixed.

7. The attachment (20) according to any of claims 1 to 6, wherein the internal layer (22a) of the separable film (22) comprises at least one of the following materials: low-density polyethylene (LDPE), very low-density polyethylene (VLDPE), polyethylene/ethyl vinyl acetate (PE/EVA).

8. The attachment (20) according to any of claims 1 to 7, wherein the external layer (22b) comprises at least one of the following materials: polyethylene (PE), polyamide (PA), poly(ethylene terephthalate) (PET).

9. The attachment (20) according to any of claims 1 to 8, wherein the attachment further comprises an additional layer of adhesive (28) fixed to the separable film (22), to the side of the external layer (22b).

10. The attachment (20) according to claim 9, wherein the additional layer of adhesive (28) is covered by a peelable protective sheet (29).

11. A surgical drape (1) comprising a main sheet (10) and a drape attachment (20) according to any of claims 1 to 10.

12. The surgical drape (1) according to claim 11, wherein the main sheet (10) is divided into two sheet parts (12, 14) which are held in extension of each other by the drape attachment (20) so as to form the main sheet (10) of said drape (1).

13. The surgical drape (1) according to claim 12, wherein the layer of adhesive (24) is applied locally in two distinct and separate zones (20A, 20B) of the separable film (22), and the drape attachment (20) is fixed to the sheet parts (12, 14) of the main sheet (10) such that each of said zones is fixed to one of the sheet parts (12, 14).

14. A process for manufacturing a surgical drape (1) according to any of claims 11 to 13, comprising the following steps:
- making a drape attachment (20) according to any of claims 1 to 10, and
- fixing the layer of adhesive (24) of the drape attachment (20) on the main sheet (10) of the surgical drape (1).

15. The manufacturing process according to claim 14, wherein the layer of adhesive (24) is applied in two distinct and separate zones of the separable film (22) during manufacture of the drape attachment (20), and the process further comprises, after the fastening step of the drape attachment (20) on said surgical drape (1), a cutting step of the main sheet (10) of the surgical drape (1) into two sheet parts (12, 14) held in extension of each other by the drape attachment (20).
